# EUROPEAN PATENT APPLICATION

(11) **EP 1 923 054 A1**
(43) Date of publication of application: **21.05.2008**
(21) Application number: 06121301.3
(22) Date of filing: 26.09.2006
(51) Int. Cl.: A61K 9/20, A61K 31/135, A61P 37/06

(54) **Fast disintegrating pharmaceutical composition comprising an S1P agonist or modulator**

(71) Applicant: Novartis AG, 4056 Basel (CH)
(72) Inventor: The designation of the inventor has not yet been filed
(74) Representative: Vögeli-Lange, Regina

(57) **Abstract**

The present invention provides a fast disintegrating solid pharmaceutical composition suitable for oral administration comprising a S1 P receptor agonist and/or modulator.

## Description

The present invention relates to pharmaceutical compositions comprising a sphingosine-1 phosphate receptor agonist and/or modulator.

Sphingosine-1 phosphate (hereinafter "S1P") is a natural serum lipid. Presently there are 8 known S1P receptors, namely S1P1 to S1P8. S1P receptor agonists have accelerating lymphocyte homing properties.

S1P receptor agonists are immunomodulating compounds which elicit a lymphopenia resulting from a re-distribution, preferably reversible, of lymphocytes from circulation to secondary lymphatic tissue, evoking a generalized immunosuppression. Naive cells are sequestered, CD4 and CD8 T-cells and B-cells from the blood are stimulated to migrate into lymph nodes (LN) and Peyer's patches (PP), and thus infiltration of cells into transplanted organs is inhibited.

The various known S1P receptor agonists show structural similarities, which result in related problems in providing a suitable formulation. In particular, there is a need for an S1P receptor agonist containing formulation which is well-adapted for oral administration in a solid form, e.g. as a tablet or capsule.

The oral route is often the most convenient route for drug administration, but unfortunately many patients have difficulties in swallowing or no water is available at the time of ingestion.

This can be overcome by using fast disintegrating tablets hence improving compliance by reducing or masking an unpleasant taste.

Accordingly, the present invention provides a fast disintegrating solid pharmaceutical composition suitable for oral administration comprising a S1P receptor agonist or modulator.

The present invention therefore provides rapid dispersing dosage forms which disintegrate rapidly in the mouth and which do not depend on the use of sweetening or flavoring agents to mask the taste nor do they depend on the presence of a liquid for washing down the dosage form.

The compositions of the present invention are capable of disintegrating in the mouth, in particular in saliva.

Preferably, the dosage forms have good mouth feel and do not exhibit premature release of the drug in the mouth.

A composition of the present invention comprises one or more disintegrants and an S1P agonist or modulator and an alkali earth metal silicate.

The ratio of the alkaline earth metal silicate:disintegration agent is from 2:1 to 10:1.

The alkaline earth metal silicates include Calcium silicate and magnesium silicate.

The disintegrants may additionally comprise effervescent agents.

Examples of disintegrants are, for example, crosscarmellose cellulose, crosspovidone and sodium starch glycolate.

The composition of the present invention may additionally comprise fillers, which may be selected from for example, gelatin, sugar alcohols, for example, mannitol, sorbitol, dextrose, sucrose, lactose, maltose, sorbitol, maltodextrins, corn syrup solids, or other saccharides or sugars, trehalose, polyvinyl pyrrolidone, polyelectrolyte gel A chondroitin sulfate, cellulose, starch derivatives, Pullulan, glycine, docusate Na, PVC, HPC-SL,mannitol & glycerol, gum xanthan/carragean/acacia/guar/tragacanth, mannitol, polysorbate 60, sodium dodecylsulfate, fatty acids, bile salts, sodium methylhydroxybenzoate, sodium propylhydroxybenzoate, polyols, and starch.

The compositions may also include, or alternatively include, lubricants e.g. magnesium stearate, calcium stearate, sodium stearyl fumarate, colloidal silica, talc.

The composition of the present invention may additionally comprise additional binders such as PVP e.g. cellulose, polyethylene glycols, polyvinylpyrrolidone, starch mucilage, acacia, alginic acid, carboxymethylcellulose, hydroxyethylcellulose, hydroxypropylcellulose, hydroxypropylmethylcellulose, dextrin, ethylcellulose, gelatin, glucose, guar gum, hydroxypropylmethylcellulose, magnesium aluminium silicate, kaltodectrin, methylcellulose, polyethylene oxide, povidone, sodium alginate and hydrogenated vegetable oils.

The core tablet compositions may also include, or alternatively include, surfactants, e.g. sodium lauryl sulphate, docusate sodium.

The core tablet compositions may also include, or alternatively include, gas producers, e.g. sodium bicarbonate, citric acid.

The composition of the present invention may additionally, or alternatively, comprise flavoring agents.

The core tablet compositions may also include, or alternatively include, glidants, e.g. silica.

The composition of the present invention may additionally, or alternatively, comprise sweeteners.

The composition of the present invention may additionally, or alternatively, comprise pH adjusting agents, e.g. citric acid, fumaric acid.

In one embodiment, there is provided a composition comprising:
0.1 to 1 % S1P agonist.
60 to 90% Filler, e.g. sugar alcohol.
20 to 45% silicate.
4 to 10% disintegrant.

Compositions of the present invention may be in the form of, for example, tablets, capsules, caplets, lozenges, pills, mini-tablets, pellets, beads and granules.

Where the solid composition is in the form of pellets or granules, these may, after application of the coating as described hereinafter, be used as such or to fill capsules, e.g. hard gelatine capsules or other storage means, for example sachets prior to administration.

Pellets and granules may be from 2mm to 0.3mm in diameter, for example, a "normal pellet" has a size of 1-0.6mm and a "bead pellet" has a size of 0.4 to 0.8mm

The composition may be held within a hydrophilic matrix which gradually or rapidly dissolves in the presence of body fluid.

The composition may additionally include materials which swell on contact with aqueous liquids, and which may be included in the composition, include polymer materials include from cross-linked sodium carboxymethylcellulose, cross-linked hydroxypropylcellulose, high molecular weight hydroxypropylcellulose, carboxymethylamide, potassium methacrylatedivinylbenzene copolymer, polymethylmethacrylate, cross-linked polyvinylpyrrolidone and high molecular weight polyvinylalcohols.

Preferably, the disintegration time (DT) of a composition of the present invention is less than 60 seconds upon contact with a fluid, e.g. water or saliva.

Particularly preferably, the DT is about 30 seconds.

Tablet hardness may be adjusted to allow any particular composition have a particular DT. In this respect, the compositions of the present invention may have varying hardness.

Accordingly, compositions of the invention may have, for example, a tensile strength of between 30N/cm² and 80N/cm².

Preferably, once disintegrated, the composition is of particle sizes from 1nm to 10mm, e.g. 50nm to 200nm, which may dissolve or may form a fine suspension.

For a fast disintegration time, the ratio of the silicate, e.g. calcium silicate to disintegrant may be from 2:1 to 10:1, for example 3:1 to 7:1, typically 6:1, 5:1 or 4:1.

In one embodiment of the present invention, the ratio of calcium silicate : disintegrant is 5:1. An example of this embodiment is a ratio of calcium silicate: Crospovidone or Croscarmellose of 5:1.

In one aspect of the present invention, the fast disintegration of the solid pharmaceutical composition increases the solubility of the active ingredient(s), for example sphingosine 1 phosphate (S1P) receptor agonists or modulators. In particular, this may be the case in saliva, leading to better solubility of the drug than in the small intestine.

In one embodiment of the present invention, there is provided a capsule containing a plurality of pellets having a fast disintegration rate according to the present invention.

The fast disintegration or the improvement in efficiency of disintegration may provide higher solubility of the active substance. Higher solubility of the drug may lead to a higher bioavailability since the risk of precipitation in the body liquid is lower.

In one embodiment of the present invention, the oral composition described herein promotes the absorption and distribution of the S1P agonist and/or modulator through the blood brain barrier and into the brain.

The bioavailability of these sphingosine 1 phosphate (S1P) receptor modulators may be improved by adding the buccal absorption site to the oral absorption site potentially leading to decrease the first-pass effect. If sphingosine 1 phosphate (S1P) receptor modulators get buccally absorbed through either the sublingual route, the oral mucosa, the esophageal lining and/or the tonsils, bioavailability would be increased as the buccal absorption route circumvents the GI tract (p-gp in the gut) and the first pass liver effect. An increased bioavailability may allow to lower the dose leading to an improved safety profile.

Pharmaceutical dosage forms adapted to supply the medicine to the oral cavity for buccal, sublingual or gingival absorption will be used with and without the presence of enhancer agents such as, but not limited to, those described in the examples.

Examples of these dosage forms are the following but not limited to: buccal spray, effervescent tablets, granules, orally disintegrating tablets, thin films or wafers and mucoadhesive discs or patches.

S1P receptor agonists are typically sphingosine analogues, such as 2-substituted 2-aminopropane-1,3-diol or 2-amino-propanol derivatives. Examples of appropriate S1P receptor agonists are, for example:

### Compounds as disclosed in EP627406A1, e.g. a compound of formula I

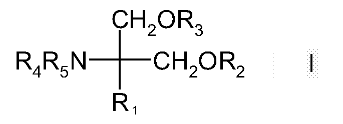

wherein
R₁ is a straight- or branched (C₁₂₋₂₂) carbon chain, which may have in the chain a bond or a hetero atom selected from a double bond, a triple bond, O, S, NR₆, wherein R₆ is H, alkyl, aralkyl, acyl or alkoxycarbonyl, and carbonyl; and/or which may have as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxyimino, hydroxy or carboxy; or
R₁ is a phenylalkyl wherein alkyl is a straight- or branched (C₆₋₂₀)carbon chain; or a phenylalkyl wherein alkyl is a straight- or branched (C₁₋₃₀)carbon chain wherein said phenylalkyl is substituted by
a straight- or branched (C₆₋₂₀)carbon chain optionally substituted by halogen,
a straight- or branched (C₆₋₂₀)alkoxy chain optionally substitued by halogen,
a straight- or branched (C₆₋₂₀)alkenyloxy;
phenylalkoxy, halophenylalkoxy, phenylalkoxyalkyl, phenoxyalkoxy or phenoxyalkyl;
cycloalkylalkyl substituted by a straight- or branched (C₆₋₂₀)alkyl chain;
heteroarylalkyl substituted by a straight- or branched (C₆₋₂₀)alkyl chain;
heterocyclic alkyl wherein said alkyl is a straight- or branched (C₆₋₂₀)carbon chain;
or
heterocyclic alkyl substituted by a straight- or branched (C₂₋₂₀)alkyl chain, and wherein the alkyl moiety may have in the carbon chain, a bond or a heteroatom selected from a double bond, a triple bond, O, S, sulfinyl, sulfonyl, or NR₆, wherein R₆ is as defined above;
and as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxy or carboxy, and
each of R₂, R₃, R₄ and R₅, independently, is H, C₁₋₄ alkyl or acyl
or a pharmaceutically acceptable salt thereof;

### Compounds as disclosed in EP 1002792A, e.g. a compound of formula II

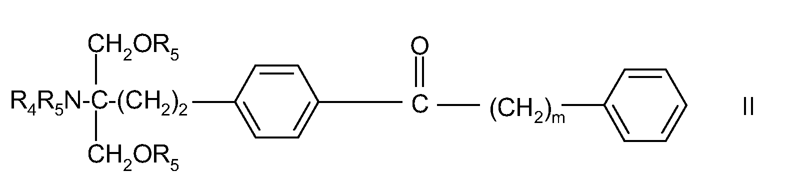

wherein
m is 1 to 9; and
each of R₂, R₃, R₄ and R₅, independently, is H, alkyl or acyl;
or a pharmaceutically acceptable salt thereof;

### Compounds as disclosed in EP0778263 A1, e.g. a compound of formula III

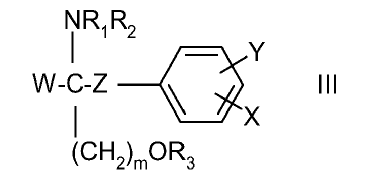

wherein
W is H; straight chain or branched (C₁₋₆)alkyl, (C₂₋₆)alkenyl or (C₂₋₆)alkynyl; unsubstituted or by OH substituted phenyl; R₄O(CH₂)ₙ; or straight chain or branched (C₁₋₆)alkyl substituted by 1 to 3 substituents selected from the group consisting of halogen, cycloalkyl, phenyl or phenyl substituted by OH;
X is H or unsubstituted or substituted straight chain alkyl having a number p of carbon atoms or unsubstituted or substituted straight chain alkoxy having a number (p-1) of carbon atoms, e.g. substituted by 1 to 3 substituents selected from the group consisting of alkyl, OH, alkoxy, acyloxy, amino, alkylamino, acylamino, oxo, haloalkyl, halogen, unsubstituted phenyl or phenyl substituted by 1 to 3 substituents selected from the group consisting of alkyl, OH, alkoxy, acyl, acyloxy, amino, alkylamino, acylamino, haloalkyl and halogen;
Y is H, alkyl, OH, alkoxy, acyl, acyloxy, amino, alkylamino, acylamino, haloalkyl or halogen, Z is a single bond or a straight chain alkylene having a number or carbon atoms of q,
each of p and q, independently, is an integer of 1 to 20, with the proviso of 6≤p+q≤23, m is 1, 2 or 3,
n is 2 or 3,
each of R₁, R₂, R₃ and R₄, independently, is H, alkyl or acyl,
or a pharmaceutically acceptable salt thereof.

### Compounds as disclosed in WO02/18395, e.g. a compound of formula IVa or IVb

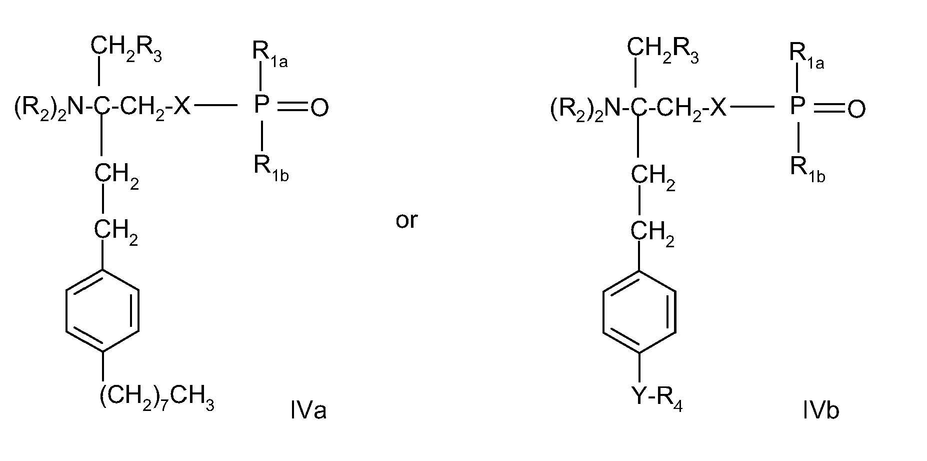

wherein
X is O, S, NR₁ or a group -(CH₂)ₙ-, which group is unsubstituted or substituted by 1 to 4 halogen;
n is 1 or 2,
R₁ is H or (C₁₋₄)alkyl, which alkyl is unsubstituted or substituted by halogen;
R₁ₐ is H, OH, (C₁₋₄)alkyl or O(C₁₋₄)alkyl wherein alkyl is unsubstituted or substituted by 1 to 3 halogen;
R_{1b} is H, OH or (C₁₋₄)alkyl, wherein alkyl is unsubstituted or substituted by halogen;
each R₂ is independently selected from H or (C₁₋₄)alkyl, which alkyl is unsubstituted or substitued by halogen;
R₃ in case of a compound of formula IVa is H, OH, halogen or O(C₁₋₄)alkyl wherein alkyl is unsubstituted or substituted by halogen;
R₃ in case of a compound of formula IVb is H, OH, halogen, (C₁₋₄)alkyl wherein alkyl is unsubstituted or substituted by hydroxy, or O(C₁₋₄)alkyl wherein alkyl is unsubstituted or substituted by halogen,
Y is -CH₂-, -C(O)-, -CH(OH)-, -C(=NOH)-, O or S,
R₄ is (C₄₋₁₄)alkyl or (C₄₋₁₄)alkenyl;
or a pharmaceutically acceptable salt or hydrate thereof;

### Compounds as disclosed in WO 02/06268 or JP-14316985, e.g. a compound of formula VII

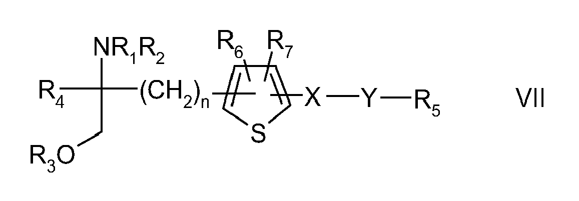

wherein
each of R₁ and R₂, independently, is H or an amino-protecting group;
R₃ is hydrogen or a hydroxy-protecting group;
R₄ is (C₁₋₆)alkyl;
n is an integer of 1-6;
X is ethylene, vinylene, ethynylene, a group having a formula - D-CH₂- (wherein, D is carbonyl, a group having a formula -CH(OH)-, O, S or N; aryl or aryl substituted by three members selected from group a as defined hereinafter;
Y is single bond, C₁₋₁₀alkylene, C₁₋₁₀alkylene which is substituted by one to three substituents selected from groups a and b, C₁₋₁₀alkylene having O or S in the middle or end of the carbon chain, or C₁₋₁₀alkylene having O or S in the middle or end of the carbon chain which is substituted by one to three substituents selected from groups a and b;
R₅ is hydrogen, cycloalkyl, aryl, heterocycle, cycloalkyl substituted by one to three members selected from groups a and b, aryl substituted by one to three members selected from groups a and b, or heterocycle substituted by one to three members selected from groups a and b; and
each of R₆ and R₇, independently, is H or a substituent selected from group a;
<group a> is halogen, lower alkyl, halogeno lower alkyl, lower alkoxy, lower alkylthio, carboxyl, lower alkoxycarbonyl, hydroxy, lower aliphatic acyl, amino, mono-lower alkylamino, di-lower alkylamino, lower aliphatic acylamino, cyano and nitro;
<group b> is cycloalkyl, aryl, heterocycle, each being optionally substituted by up to three substituents selected from group a;
with the proviso that when R₅ is hydrogen, Y is either a single bond or linear C₁₋₁₀ alkylene,
e.g. (2R)-2-amino-4-[3-(4-cyclohexyloxybutyl)benzo[b]thien-6-yl]-2-methylbutan-1-ol,
or a pharmacologically acceptable salt or ester thereof.

When in the compounds of formula I the carbon chain as R₁ is substituted, it is preferably substituted by halogen, nitro, amino, hydroxy or carboxy. When the carbon chain is interrupted by an optionally substituted phenylene, the carbon chain is preferably unsubstituted. When the phenylene moiety is substituted, it is preferably substituted by halogen, nitro, amino, methoxy, hydroxy or carboxy. Acyl may be a residue R-CO-, wherein R is C₁₋₆alkyl, C₃₋₆cycloalkyl, phenyl or phenyl-C₁₋₄alkyl.

Preferred compounds of formula I are those wherein R₁ is a straight or branched, preferably straight, chain alkyl having 13-20 carbon atoms, optionally substituted by nitro, halogen, amino, hydroxy or carboxy, and, more preferably those wherein R₁ is phenylalkyl substituted by a straight or branched C₆₋₁₄alkyl chain optionally substituted by halogen and the alkyl moiety is a C₁₋₆alkyl optionally substituted by hydroxy. More preferably, R₁ is phenyl-C₁₋₆alkyl substituted on the phenyl by a straight or branched, preferably straight, C₆₋₁₄alkyl chain. The C₆₋₁₄alkyl chain may be in ortho, meta or para, preferably in para.

Preferably each of R₂ to R₅ is H.

A preferred compound of formula I is 2-amino-2-tetradecyl-1,3-propanediol. A particularly preferred S1P receptor agonist of formula I is 2-amino-2-[2-(4-octylphenylethyl)]propane-1,3-diol in free form or in a pharmaceutically acceptable salt form (referred to hereinafter as Compound A), e.g. the hydrochloride, i.e. FTY720, as shown:

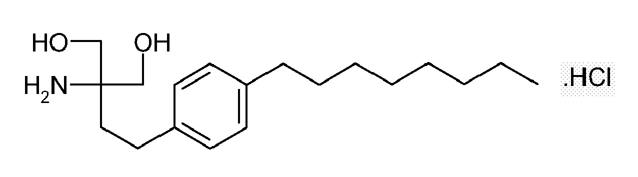

A preferred compound of formula II is one wherein each of R₂ to R₅ is H and m is 4, i.e. 2-amino-2-{2-[4-(1-oxo-5-phenylpentyl)phenyl]ethyl}propane-1,3-diol (referred to hereinafter as Compound B), in free form or in a pharmaceutically acceptable salt form, e.g. the hydrochloride.

A preferred compound of formula IVa is the Compound A-phosphate (R₂ is H, R₃ is OH, X is O, R₁ₐ and R_{1b} are OH). A preferred compound of formula V is Compound B-phosphate (R₁ is CH₂OH, R₃ is H, X is O, m is 1, R₂ is phosphate and R is 2-[4-(1-oxo-5-phenylpentyl)phenyl]ethyl).

When the compounds of formulae I to VII have one or more asymmetric centers in the molecule, the various optical isomers, as well as racemates, diastereoisomers and mixtures thereof are embraced.

Examples of pharmaceutically acceptable salts of the compounds of formulae I to VII include salts with inorganic acids, such as hydrochloride, hydrobromide and sulfate, salts with organic acids, such as acetate, fumarate, maleate, benzoate, citrate, maleate, methanesulfonate and benzenesulfonate salts, or, when appropriate, salts with metals, such as sodium, potassium, calcium and aluminium, salts with amines, such as triethylamine and salts with dibasic amino acids, such as lysine. The compounds and salts of the present invention encompass hydrate and solvate forms.

The composition of the present invention may comprise one or more salts and/or free acid of the S1 P agonists and//or modulator.

The composition of the invention preferably contains 0.01 to 20% by weight of S1 P receptor agonists, more preferably 0.1 to 10%, e.g. 0.5 to 5% by weight, based on the total weight of the composition.

Preferably, the composition is stable for at least six months at ambient temperature.

Preferably, the active ingredient dose ranges from 0 to 1000 mg, for example 0 to 500mg.

The compositions of the invention may show good stability characteristics as indicated by standard stability trials, for example having a shelf life stability of up to one, two or three years, and even longer. Stability characteristics may be determined, e.g. by measuring decomposition products by HPLC analysis after storage for particular times, at particular temperatures, e.g. 20°, 40° or 60°C.

The pharmaceutical compositions of the present invention may be produced by standard processes, for instance by conventional mixing, granulating, sugar-coating, dissolving or lyophilizing processes. Procedures which may be used are known in the art, e.g. those described in L. Lachman et al. The Theory and Practice of Industrial Pharmacy, 3rd Ed, 1986, H. Sucker et al, Pharmazeutische Technologie, Thieme, 1991, Hagers Handbuch der pharmazeutischen Praxis, 4th Ed. (Springer Verlag, 1971) and Remington's Pharmaceutical Sciences, 13th Ed., (Mack Publ., Co., 1970) or later editions.

In particular, the pharmaceutical compositions are useful for:
a) treatment and prevention of organ or tissue transplant rejection, for example for the treatment of the recipients of heart, lung, combined heart-lung, liver, kidney, pancreatic, skin or corneal transplants, and the prevention of graft-versus-host disease, such as sometimes occurs following bone marrow transplantation; particularly in the treatment of acute or chronic allo- and xenograft rejection or in the transplantation of insulin producing cells, e.g. pancreatic islet cells;
b) treatment and prevention of autoimmune disease or of inflammatory conditions, e.g. multiple sclerosis, arthritis (for example rheumatoid arthritis), inflammatory bowel disease, hepatitis, etc.;
c) treatment and prevention of viral myocarditis and viral diseases caused by viral mycocarditis, including hepatitis and AIDS.

The invention is, in one embodiment, related to the treatment of inflammatory conditions. In one example, the invention is related to compositions capable of regulating and/or suppressing mast cell activation and secretion for the relief of inflammatory conditions, e.g., in the brain as in multiple sclerosis.

There is also provided a method of protecting multiple sclerosis subjects against neurodegerative brain inflammation, comprising the administration to said subjects the a composition as described herein, for example a composition comprising an S1 P agonist and/or modulator.

The composition of the present invention and any concentrate for dilution and pharmaceutical solution made therefrom, may be administered in an amount which is therapeutically effective against a disease or condition which can be treated by administration of the S1 P receptor agonist.

The exact amount of S1P receptor agonist or pharmaceutically acceptable salt thereof to administer can vary widely. The dose may depend on the particular compound, route of administration, the rate of administration, the strength of the particular concentrate or pharmaceutical solution employed, the nature of the disease or condition being treated, and the sex, age and body weight of the patient. The dose may also depend on the existence, nature and extent of any adverse side-effects that may accompany the administration of the concentrate or pharmaceutical formulation. Typically, a dose of 0.5 to 5 mg of S1 P receptor agonist, e.g. Compound A, are administered to children.

The composition of the present invention and any concentrate for dilution and respective pharmaceutical solution may be used in combination with other immunosuppressant(s), steroid(s) such as prednisolone, methylprednisolone, dexamethasone, hydrocortisone and the like, or nonsteroidal antiinflammatory agent. The administration of a combination of active agents may be simultaneous or consecutive, with either one of the active agents being administered first. The dosage of the active agents of a combination treatment may depend on effectiveness and site of action of each active agent, as well as synergistic effects between the agents used for combination therapy.

The invention will now be described with reference to the following specific embodiments.

### Examples

### Example 1

An example for a 7 mm round, 127 mg tablet for fast disintegration according to the present invention:

| Ingredient | mg/dose |
|---|---|
| FTY720 HCl | 0.56 |
| Directly compressible mannitol, e.g. Parteck M200 | 82.54 |
| Calcium silicate | 36.00 |
| Magnesium stearate | 0.90 |
| Crospovidone | 7.00 |

The tablet may be manufactured by known methods. For example, the tablet may be manufactured by blending of all ingredients and further compressing to tablets and/or granulation and/or micronisation and further compressing of the granules to tablets.

## Claims

**1.** A fast disintegrating solid pharmaceutical composition comprising:
(a) an S1P agonist or modulator
(b) an alkaline earth metal silicate
(c) a disintegration agent
wherein the ratio of the silicate:disintegration agent is from 2:1 to 10:1

**2.** The composition of claim 1, where the ratio is 3:1 to 7:1.

**3.** The composition of claim 2, where the ratio is 5:1.

**4.** The composition of claim 1, 2 or 3, wherein the disintegration agent is selected from Crospovidone and Croscarmellose.

**5.** A composition according to any preceding claim, wherein the S1P receptor agonist comprises 2-amino-2-[2-(4-octylphenyl)ethyl]propane-1,3-diol or 2-amino-2-{2-[4-(1-oxo-5-phenylpentyl)-phenyl]ethyl}propane-1,3-diol or a pharmaceutically acceptable salt thereof.

**6.** A composition according any preceding claim, further comprising a lubricant.

**7.** A composition according to claim 6, wherein the lubricant comprises magnesium stearate.

**8.** A composition according to any preceding claim, comprising 0.5 to 5% by weight of the S1P receptor agonist.

**9.** A composition according to claim 6 or 7, comprising 1.5 to 2.5% by weight of the lubricant.

**10.** A composition according to any preceding claim, further comprising hydroxypropyl cellulose, hydroxypropylmethyl cellulose or methyl cellulose.

**11.** A composition according to any preceding claim, in the form of a tablet.

**12.** A composition according to any preceding claim, wherein the disintegration time is less than 60 seconds.

**13.** A method of treating a subject in need of immunosuppression, comprising administering to the subject a composition according to any of claims 1 to 12.

**15.** Use of a pharmaceutical composition according to any of claims 1 to 12 for the preparation of a medicament for the prevention or treatment of organ or tissue transplant rejection, or for the prevention or treatment of an inflammatory or autoimmune disease.

**16.** A method of protecting multiple sclerosis subjects against neurodegerative brain inflammation, comprising the administration to said subjects the composition of any of claims 1 to 12.
